# EUROPEAN PATENT APPLICATION

(11) **EP 2 656 856 A2**
(43) Date of publication of application: **30.10.2013**
(21) Application number: 13002069.6
(22) Date of filing: 19.04.2013
(51) Int. Cl.: A61K 47/00, A61K 9/20, A61K 9/24, A61K 31/195

(54) **Tablet formulation comprising levodopa, carbidopa, entacapone providing extended release**

(30) Priority: 25.04.2012 TR 201204839
(71) Applicant: Ali Raif Ilaç Sanayi ve Ticaret Anonim Sirketi, 34418 Istanbul (TR)
(72) Inventor: Bulgur, Abdullah, Istanbul (TR); Kesgin, Didehan, Istanbul (TR)
(74) Representative: Bulut, Pinar

(57) **Abstract**

The present invention is related to a composition intended for the treatment of Parkinson disease which comprises levodopa, carbidopa, entacapone or pharmaceutically acceptable salts, solvates or hydrates of these molecules in a combined form. The invention is characterized in that it comprises both levodopa and carbidopa as extended release form and entacapon as immediately release form.

## Description

### Technical Aspect:

The present invention is related to a composition intended for the treatment of Parkinson disease which comprises levodopa, carbidopa, entacapone or pharmaceutically acceptable salts, solvates or hydrates of these molecules in a combined form. The invention is characterized in that it comprises levodopa and carbidopa as extended release form and entacapon as immediately release form.

### Prior Art:

The chemical name of Levodopa (Formula I), which is the metabolic precursor of dopamine, is (S)-2-amino-3-(3,4-dihydroxyphenyl)propanoic acid. Levodopa is an aromatic amine whose molecular weight is 197.19 g/mol and metabolized with dopamine. The molecule's closed formula is C₉H₁₁NO₄.

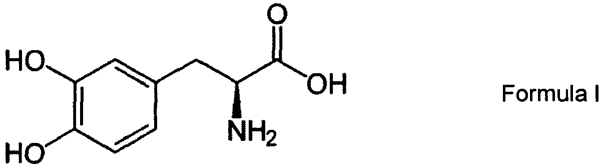

Carbidopa (Formula II) is a peripheric decarboxylase inhibitor whose molecular weight is 226.229 g/mol. When administered alone, it does not show a pharmaceutical activity. Its chemical name is (2*S*)-3-(3,4-dihydroxyphenyl)-2-hydrazino-2-methylpropanoic acid. The molecule's closed formula is C₁₀H₁₄N₂O₄.

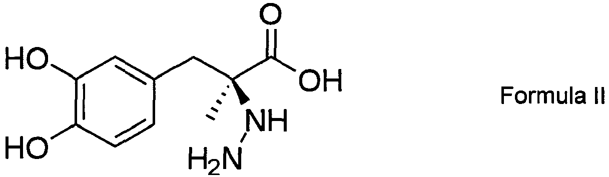

Entacapone (Formula III) is a catechol-o-methyltransferase (COMT) inhibitor used in Parkinson treatment. Its molecular weight is 305.286 g/mol. Its chemical name is (2*E*)-2-cyano-3-(3,4-dihydroxy-5-nitrophenyl)-*N*,*N*-diethylprop-2-eneamide. Its closed formula is C₁₄H₁₅N₃O₅.

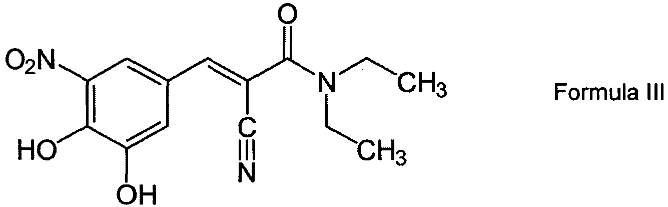

In European market, there are commercial products called COMTESS and COMTAN which comprise Entacapone and manufactured by Orion-Finland. Drugs containing levodopa and carbidopa are the most frequently used ones for the treatment of Parkinson disease. Drugs containing levodopa and carbidopa are sold under various trademarks such as Sinemet, Parcopa and Atamet.

Products containing extended release Levodopa, Carbidopa, Entacapone are not present in the market. There are no study present in the literature related to formulations comprising extended release levodopa and carbidopa and immediately release entacapone. There are studies present in the literature which are related to immediately release tablets and formulations comprising extended release levodopa and carbidopa in the form of triple combinations.

There are various patents related to drugs containing levodopa, carbidopa and entacapone. In patents US 6,500,867 (Orion) and US 6,797,732 (Orion), pharmaceutical compositions which comprise levodopa, carbidopa and entacapone are described. In both patents, it is stated that there are stability problems and the desired therapeutic effect can not be obtained when entacapone is mixed with levodopa and carbidopa.

Moreover, it is also stated that more stable results and therapeutic efficiency are obtained when a major part of carbidopa is separated from levodopa and entacapone.

The characteristic of US 6,500,867 (ORION) is that, a major part of carbidopa is seperated from entacapone and levodopa. The characteristic of US 6,797,732 (Orion) is that, it does not contain microcrystal cellulose.

In PCT application WO 2008/053297 A2 (WOCKHARDT), a formulation is described in which a major part of entacapon is seperated from levodopa and carbidopa mixture.

In PCT application WO 2010/027340 A1 (Dr.FRIK), a formulation which comprises the mixture of levodopa granule and carbidopa with entacapone granule is described.

In US5840756 (TEVA), a matrix, which comprises hydroxypropylmethylcellulose, hydroxypropylcellulose and carboxyviniyl polymer for the extended release of levodopa, is used.

In patent application EP0253490 A1 (MERCK Co.), a levodopa and carbidopa formulation dispersed in a polymeric matrix, which is composed of a mixture of a water soluble polymer selected among hydroxypropylcellulose, hydroxypropylmethyl cellulose, polyvinyl pyrolidone, polyethylene glycol, starch and methyl cellulose and two poorly water soluble polymer selected among polyvinyl acetate/crotonic acid copolymer, polyvinyl chloride, polyethylene, cellulose acetate, polyvinyl alcohol, ethylene vinylacetate copolymer, polyvinyl acetate, polymethylmethacrylate and ethyl cellulose, is explained.

Patent application EP1262198 A1 is related to a pharmaceutical composition which is dispersed in hydroxypropylmethyl cellulose as an effective amount of hydrophilic matrix and which comprises an organic acid such as fumaric acid and citric acid.

### Description of the Invention:

The present invention is related to a composition intended for the treatment of Parkinson disease which comprises levodopa, carbidopa, entacapone or pharmaceutically acceptable salts, solvates or hydrates thereof in a combined form.

In the present invention, a formulation is explained in which the entacapone or pharmaceutically acceptable salt or hydrate thereof is separated from the mixture of levodopa and carbidopa or pharmaceutically acceptable salts or hydrates thereof. The invention is characterized in that it comprises levodopa and carbidopa as extended release form and entacapone as immediately release form.

The present invention is a matrix type formulation in which polyethylene oxide polymer is selected as the polymer which control the release by forming gel. The molecular weight of the polyethylene oxide polymer used is 7,000,000 g/mol and Brookfield viscosity value of its 1% m/v aqueous solution at 25°C is 7500-10000 cps (Colorcon - Sentry Polyox WSR 303 - Leo - NF - Dow).

The present invention is related to a solid, stable, double layer composition which comprises hydroxypropylcellulose (Hercules, Klucel) with polyethylene oxide polymer that forms a gel as a release controlling agent and also comprises an organic acid and whose portion that comprises levodopa and carbidopa is long acting and other portion that comprises entacapone is immediately release.

Another characteristic of the present invention is that, it comprises organic acids such as fumaric acid and citric acid for stabilization.

In the present invention a double layer tablet formulation is described. One of the layers comprises immediately release entacapone or pharmaceutically acceptable salts or hydrates thereof and the other layer comprises the mixture of extended release levodopa and carbidopa or pharmaceutically acceptable salts or hydrates thereof.

The first layer of the double layer tablet comprises Levodopa and Carbidopa as active ingredient, both polyethylene oxide and hydroxypropyl cellulose as release controlling agents, colloidal silicone dioxide as glidant, citric acid and/or fumaric acid as stabilizing agent and magnesium stearate as lubricant.

On the other hand, the second layer of the double layer tablet comprises entacapone as active ingredient, microcrystalline cellulose as dispersing and diluent, mannitol as diluent, crosscarmellose sodium as disintegrant, polysorbate 80 as surfactant and magnesium stearate as lubricant.

### EXAMPLES :

**Table 1. Test formulations (quantities are given in mg)**

| **First layer** | mg |
|---|---|
| Levodopa | 200.0 |
| Carbidopa monohydrate | 53.5 |
| Polyethylene oxide | 90.0 |
| Hydroxypropylcellulose | 37.1 |
| Colloidal silicon dioxide | 2.4 |
| Fumaric acid | 10.0 |
| Magnesium stearate | 7.0 |
| Total | 400.0 |

| **Second layer** | |
|---|---|
| Entacapone | 200.0 |
| Microcrystalline cellulose | 50.0 |
| Mannitol | 20.0 |
| Croscarmellose sodium | 15.0 |
| Polysorbate 80 | 1.0 |
| Magnesium stearate | 1.0 |
| Total | 287.0 |
| **Total tablet weight** | **687.0** |

The prepared tablets are coated with a suitable coating agent in such a way that their weight increases 3%.

The prepared formulation is tested for carbidopa and levodopa with the dissolution method present in FDA of Sinemet SR, which is the original product, and it is compared with the original product. According to the dissolution specifications given by FDA; paddle method is used at 0.1 N HCl, at 50 rpm with 900 ml volume, samples were taken in 30th, 60th, 150th and 240th minutes and f2 value (similarity factor) was calculated and the original product and the prepared product was found similar.

The prepared formulation was studied comparatively with the original product Stalevo 200 under the dissolution conditions given by FDA. According to the dissolution specifications given by FDA; basket method is used for entacapone at pH 5.5 phosphate buffer, at 125 rpm, with 900ml volume and f2 value (similarity factor) was calculated and the original product and the prepared product was found similar.

The above examples describe the invention, but do not limit it in any way.

## Claims

1. A double layer tablet comprising levodopa, carbidopa, entacapone or pharmaceutically acceptable salts, solvates or hydrates thereof, **characterized in that** the first layer contains extended release levodopa and carbidopa and the second layer contains immediately release entacapone.

2. A double layer tablet according to Claim 1, wherein it comprises polyethylene oxide polymer as release controlling agent in the extended release layer.

3. A double layer tablet according to Claim 1, wherein the ratio of polyethylene oxide polymer present in the extended ralease layer is 20-30% by weight of the extending release layer.

4. A double layer tablet according to Claim 1, wherein it comprises both polyethylene oxide polymer and hydroxypropylcellulose as release controlling agents.

5. A double layer tablet according to previous claims, wherein the molecular weight of polyethylene oxide polymer is 7,000,000 g/mol and the Brookfield viscosity value of its 1% m/v aqueous solution at 25°C is 7500-10000 cps.

6. A double layer tablet according to previous claims, wherein it comprises organic acid in the extended release layer.

7. A double layer tablet according to claim 6, wherein organic acid is citric acid or fumaric acid.

8. A double layer tablet according to previous claims, wherein the extended release layer comprises levodopa and carbidopa as active ingredient, both polyethylene oxide and hydroxypropyl cellulose as release controlling agents, colloidal silicone dioxide as glidant, citric acid and fumaric acid as stabilizing agents and magnesium stearate as lubricant.

9. A double layer tablet according to previous claims, wherein the immediately release layer comprises entacapone as active ingredient, microcrystalline cellulose as dispersing agent and diluent, mannitol as diluent, crosscarmellose sodium as disintegrant, polysorbate 80 as surfactant and magnesium stearate as lubricant.

10. A double layer tablet according to preceding claims, wherein it is coated with a suitable coating.

## Amended claims

### Amended claims in accordance with Rule 137(2) EPC.

**1.** A double layer tablet comprising levodopa, carbidopa, entacapone or pharmaceutically acceptable salts, solvates or hydrates thereof, **characterized in that** the first layer contains extended release levodopa and carbidopa and the second layer contains immediately release entacapone, wherein it comprises polyethylene oxide polymer as release controlling agent in the extended release layer.

**2.** A double layer tablet according to Claim 1, wherein the ratio of polyethylene oxide polymer present in the extended release layer is 20-30% by weight of the extending release layer.

**3.** A double layer tablet according to Claim 1, wherein it comprises both polyethylene oxide polymer and hydroxypropylcellulose as release controlling agents.

**4.** A double layer tablet according to previous claims, wherein the molecular weight of polyethylene oxide polymer is 7,000,000 g/mol and the Brookfield viscosity value of its 1% m/v aqueous solution at 25°C is 7500-10000 cps.

**5.** A double layer tablet according to previous claims, wherein it comprises organic acid in the extended release layer.

**6.** A double layer tablet according to claim 6, wherein organic acid is citric acid or fumaric acid.

**7.** A double layer tablet according to previous claims, wherein the extended release layer comprises levodopa and carbidopa as active ingredient, both polyethylene oxide and hydroxypropyl cellulose as release controlling agents, colloidal silicone dioxide as glidant, citric acid and fumaric acid as stabilizing agents and magnesium stearate as lubricant.

**8.** A double layer tablet according to previous claims, wherein the immediately release layer comprises entacapone as active ingredient, microcrystalline cellulose as dispersing agent and diluent, mannitol as diluent, crosscarmellose sodium as disintegrant, polysorbate 80 as surfactant and magnesium stearate as lubricant.

**9.** A double layer tablet according to preceding claims, wherein it is coated with a suitable coating.
